# EUROPEAN PATENT APPLICATION

(11) **EP 4 549 467 A2**
(43) Date of publication of application: **07.05.2025**
(21) Application number: 25151614.2
(22) Date of filing: 26.07.2017
(51) Int. Cl.: C07K 16/28

(54) **ADMINISTRATION OF HYPOXIA ACTIVATED PRODRUGS IN COMBINATION WITH IMMUNE MODULATORY AGENTS FOR TREATING CANCER**

(30) Priority: 01.08.2016 US 201662369691 P
(62) Divisional of application: 17837425.2
(71) Applicant: ImmunoGenesis, Inc., Houston, TX 77010 (US)
(72) Inventor: HART, Charles, P., Menlo Park, 94025 (US)
(74) Representative: HGF

(57) **Abstract**

Cancer can be treated by administration of TH-302 in combination with an immunomodulator.

## Description

### CROSS-REFERENCE TO RELATED APPLICATIONS

This application claims benefit of priority of U.S. Provisional Application No. 62/369,691 filed on August 1, 2016 of which is incorporated herein by reference in its entirety.

### FIELD OF THE INVENTION

The present invention provides methods for treating cancer, and pharmaceutical formulations and unit dose forms useful in those methods. The invention therefore relates to the fields of medicine and pharmacology.

### BACKGROUND OF THE INVENTION

Certain agents have been made for treating cancer by targeting hypoxic cancer cells (see e.g. for example, U.S. Patent Nos. 7,550,496; 8,299,088; 8,003,625; 8,507,464; 8,664,204; 9,226,932; 8,552,048; and 8,946,275 and U.S. Patent Application Nos. 13/806,088; 13/809,135; 14/009,068; 14/110,819; 14/033491; 14/367,188; 14/367,152; 14/375,417; 14/380,054; 14/783776; and 14/907,190 and PCT International Patent Application Nos. PCT/US2014/062532; PCT/US2015/029297; PCT/US2015/040642; PCT/US15/41100; and PCT/US2015/61248 each of which is incorporated herein by reference) which disclose hypoxia-activated prodrugs (HAP)s. One such example of such a HAP is TH-302 or evofosfamide.

Many cancers protect themselves from the immune system by inhibiting the T cell signal. CTLA-4 (Cytotoxic T-Lymphocyte-Associated protein 4, aka CD152), PD-1 (Programmed Death 1) and KIR (Killer-cell Immunoglobulin-like Receptor) receptors are immune checkpoints that also down regulate the immune system. See Noman et al. J. Exp. Med. 211(5): 781-790 (2014); Corzo et al. J. Exp. Med. 207(11): 2439-2453 (2010); Marotta et al. Cancer Res., 71(3): 779-789 (2011), incorporated herein by reference. In recent years, CTLA-4 and PD-1 inhibitors have been increasingly considered as new targets for cancer immunotherapies due to the effectiveness of two checkpoint inhibitor drugs that were initially indicated for advanced melanoma. Ipilimumab (trade name Yervoy), is a monoclonal antibody that works to activate the immune system by targeting CTLA-4. Pembrolizumab (trade name Keytruda) is a human antibody used in cancer immunotherapy targeting the PD-1 receptor. PD-1 has two ligands, PD-L1 and PD-L2. Nivolumab, (tradename Opdivo) is a human IgG4, anti-PD-1 monoclonal antibody developed to activate the immune system by targeting PD-L1. Another strategy is to target agonists of immune checkpoints that up regulate the immune system, e.g. the 4-1BB receptor (CD137). Agonistic 4-1BB monoclonal antibodies work to activate the immune system by targeting 4-1BB. No 4-1BB agonist has been approved for cancer or immune treatment alone or in combination with another anti-cancer agent.

Despite the success of T cell immune checkpoint blockade in treating melanoma, other carcinomas, e.g. adenocarcinomas of the prostate and pancreas are largely resistant to CTLA-4 and PD-1 antibody therapy in animal models and in man. The hypoxic zones of these tumors resist infiltration by T cells even in the context of robust infiltration of T cells in normoxic areas of the same tumor (e.g. in the context of T cell checkpoint blockade). Therefore, there remains a need for more effective and safer cancer therapies. The current invention provides such therapies.

### SUMMARY OF THE INVENTION

In a first aspect, the present invention provides a method of treating cancer, said method comprising administering to a patient in need of such treatment a therapeutically effective amount of a HAP in combination with a therapeutically effective amount of an immune modulatory agent or immunomodulator, such as an anti-CTLA-4, anti-PD-1, anti-PD-L1, anti-PD-L2 antibody or an agonistic 4-1BB antibody, including but not limited to ipilimumab, pembrolizumab and nivolumab.

In various embodiments, the HAP is the compound known as TH-302 shown below; and a pharmaceutically acceptable carrier, excipient, or diluent. TH-302 is converted to the alkylating agent/nitrogen mustard compound Br-IPM as shown below *in vivo:*

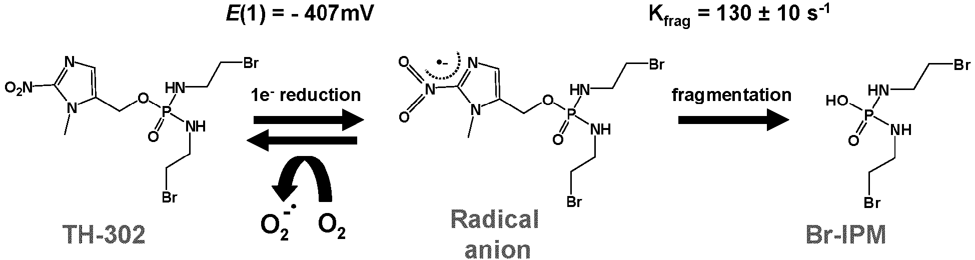

In various embodiments, the TH-302 is administered at a dose and frequency described in U.S. Patent Nos. 7,550,496; 8,299,088; 8,003,625; 8,507,464; 8,664,204; 9,226,932; 8,552,048; and 8,946,275 and U.S. Patent Application Nos. 13/806,088; 13/809,135; 14/009,068; 14/110,819; 14/033491; 14/367,188; 14/367,152; 14/375,417; 14/380,054; 14/783776; and 14/907,190 and PCT International Patent Application Nos. PCT/US2014/062532; PCT/US2015/029297; PCT/US2015/040642; PCT/US15/41100; and PCT/US2015/61248, which are incorporated herein by reference.

In one embodiment, the present invention provides a method of increasing antitumor effect of a HAP on a patient, comprising co-administering to the tumor cell (or to a patient with a tumor) an immunomodulator in combination with a HAP. As used herein, "increasing the antitumor effect" of a HAP by co-administration of an immunomodulator refers to one or more of (i) increasing the number of tumor cells killed by the HAP relative to the number that would be killed in the absence of co-administration of an immunomodulator; or (ii) overcoming a tumor cell's resistance to an alkylator. Co-administration, as used herein, contemplates that the two drugs co-administered exert their pharmacological effect in a tumor cell at the same time; such co-administration can be achieved by simultaneous, contemporaneous, or sequential administration of the two drugs. In one embodiment, the immunomodulator is ipilimumab, pembrolizumab or nivolumab. In another embodiment, the HAP is TH-302. In one embodiment, TH-302 is administered once weekly for five weeks. In other embodiments, TH-302 is administered no more than once a week. Within these embodiments, in some embodiments, TH-302 therapy is continued for multiple weeks or in some embodiments, TH-302 is not administered for some weeks of the multiple week cycle.

In a second aspect, the present invention provides pharmaceutical formulations and unit dose forms suitable for use in the methods of the present invention. In one embodiment, the hypoxia activated prodrug and the immunomodulator are formulated separately in distinct unit dose forms. In another embodiment, the hypoxia activated prodrug and the immunomodulator are formulated together in an admixture or other combination pharmaceutical formulation and combination unit dose forms. In various embodiments, the hypoxia activated prodrug in the formulation and unit dose forms is TH-302.

### DETAILED DESCRIPTION OF THE INVENTION

The practice of the present invention includes the use of conventional techniques of organic chemistry, molecular biology (including recombinant techniques), microbiology, cell biology, biochemistry and immunology, which are within the skill of the art.

### Definitions

In this specification and in the claims that follow, reference will be made to a number of terms that have the meanings below. All numerical designations, e.g., pH, temperature, time, concentration, and weight, including ranges of each thereof, are approximations that typically may be varied ( + ) or ( - ) by increments of 0.1, 1.0, or 10.0, as appropriate. All numerical designations may be understood as preceded by the term "about". Reagents described herein are exemplary and equivalents of such may be known in the art.

The singular form "a", "an", and "the" includes plural references unless the context clearly dictates otherwise.

The term "comprising" means any recited elements are necessarily included and other elements may optionally be included. "Consisting essentially of" means any recited elements are necessarily included, elements that would materially affect the basic and novel characteristics of the listed elements are excluded, and other elements may optionally be included. "Consisting of" means that all elements other than those listed are excluded. Embodiments defined by each of these terms are within the scope of this invention.

Certain compounds utilized in the present invention possess asymmetric carbon atoms (optical centers) or double bonds; the racemates, diastereomers, geometric isomers, regioisomers and individual isomers (e.g., separate enantiomers) are all intended to be encompassed within the scope of the present invention. The compounds of the present invention may also contain unnatural proportions of atomic isotopes at one or more of the atoms that constitute such compounds. For example, the compounds may be radiolabeled with radioactive isotopes, such as for example, and without limitation, tritium (³H), iodine-125 (¹²⁵I), or carbon-14 (¹⁴C). All isotopic variations of the compounds of the present invention, whether radioactive or not, are intended to be encompassed within the scope of the present invention.

Other terms related to this invention are defined below.

"Administering" or "administration of" a drug to a patient (and grammatical equivalents of this phrase) refers to direct administration, which may be administration to a patient by a medical professional or may be self-administration, and/or indirect administration, which may be the act of prescribing a drug. For example, a physician who instructs a patient to self-administer a drug and/or provides a patient with a prescription for a drug is administering the drug to the patient.

"Cancer" refers to malignant solid tumors of potentially unlimited growth, as well as various blood cancers that may originate from cancer stem cells in the bone marrow, which can expand locally by invasion and systemically by metastasis. Examples of cancers include, but are not limited to, cancer of the adrenal gland, bone, brain, breast, bronchi, colon and/or rectum, gallbladder, gastrointestinal tract, head and neck, kidneys, larynx, liver, lung, neural tissue, pancreas, prostate, parathyroid, skin, stomach, and thyroid. Other examples of cancers include, adenocarcinoma, adenoma, basal cell carcinoma, cervical dysplasia and in situ carcinoma, Ewing's sarcoma, epidermoid carcinomas, giant cell tumor, glioblastoma multiforma, hairy-cell tumor, intestinal ganglioneuroma, hyperplastic corneal nerve tumor, islet cell carcinoma, Kaposi's sarcoma, leiomyoma, leukemias, lymphomas, malignant carcinoid, malignant melanomas, malignant hypercalcemia, marfanoid habitus tumor, medullary carcinoma, metastatic skin carcinoma, mucosal neuroma, myelodisplastic syndrome, myeloma, mycosis fungoides, neuroblastoma, osteosarcoma, osteogenic and other sarcoma, ovarian tumor, pheochromocytoma, polycythermia vera, primary brain tumor, small-cell lung tumor, squamous cell carcinoma of both ulcerating and papillary type, seminoma, soft tissue sarcoma, retinoblastoma, rhabdomyosarcoma, renal cell tumor or renal cell carcinoma, veticulum cell sarcoma, and Wilm's tumor. Examples of cancers also include astrocytoma, a gastrointestinal stromal tumor (GIST), a glioma or glioblastoma, renal cell carcinoma (RCC), hepatocellular carcinoma (HCC), and a pancreatic neuroendocrine cancer.

"Combination therapy" or "combination treatment" refers to the use of two or more drugs in therapy, i.e., use of a hypoxia activated prodrug as described herein together with one or more immunomodulators to treat cancer. Administration in "combination" refers to the administration of two or more agents (e.g., a hypoxia activated prodrug and an immunomodulator, and optionally one or more anti-cancer agents, for treating cancer) in any manner in which the pharmacological effects of both are manifest in the patient at the same time. Thus, administration in combination does not require that a single pharmaceutical composition, the same dosage form, or the same route of administration be used for administration of both agents or that the two agents be administered at precisely the same time. For example, and without limitation, it is contemplated that an immunomodulator can be administered with a hypoxia activated prodrug in accordance with the present invention as a combination therapy.

"Hyperproliferative disease" refers to a disease characterized by cellular hyperproliferation (e.g., an abnormally increased rate or amount of cellular proliferation). Cancer is a hyperproliferative disease. Examples of hyperproliferative diseases other than cancer include, but are not limited to, allergic angiitis and granulomatosis (Churg-Strauss disease), asbestosis, asthma, atrophic gastritis, benign prostatic hyperplasia, bullous pemphigoid, coeliac disease, chronic bronchitis and chronic obstructive airway disease, chronic sinusitis, Crohn's disease, demyelinating neuropathies, dermatomyositis, eczema including atopic dermatitis, eustachean tube diseases, giant cell arteritis, graft rejection, hypersensitivity pneumonitis, hypersensitivity vasculitis (Henoch-Schonlein purpura), irritant dermatitis, inflammatory hemolytic anemia, inflammatory neutropenia, inflammatory bowel disease, Kawasaki's disease, multiple sclerosis, myocarditis, myositis, nasal polyps, nasolacrimal duct diseases, neoplastic vasculitis, pancreatitis, pemphigus vulgaris, primary glomerulonephritis, psoriasis, periodontal disease, polycystic kidney disease, polyarteritis nodosa, polyangitis overlap syndrome, primary sclerosing cholangitis, rheumatoid arthritis, serum sickness, surgical adhesions, stenosis or restenosis, scleritis, scleroderma, strictures of bile ducts, strictures (of duodenum, small bowel, and colon), silicosis and other forms of pneumoconiosis, type I diabetes, ulcerative colitis, ulcerative proctitis, vasculitis associated with connective tissue disorders, vasculitis associated with congenital deficiencies of the complement system, vasculitis of the central nervous system, and Wegener's granulomatosis.

"Hypoxia activated prodrug" refers to a drug that is less active or inactive under normoxia than under hypoxia or anoxia. Hypoxia activated prodrugs include drugs that are activated by a variety of reducing agents, including without limitation single electron transferring enzymes (such as cytochrome P450 reductases) and two electron transferring (or hydride transferring) enzymes (see U.S. Patent Nos. 7,550,496; 8,299,088; 8,003,625; 8,507,464; 8,664,204; 9,226,932; 8,552,048; and 8,946,275 and U.S. Patent Application Nos. 13/806,088; 13/809,135; 14/009,068; 14/110,819; 14/033491; 14/367,188; 14/367,152; 14/375,417; 14/380,054; 14/783776; and 14/907,190 and PCT International Patent Application Nos. PCT/US2014/062532; PCT/US2015/029297; PCT/US2015/040642; PCT/US15/41100; and PCT/US2015/61248, each of which is incorporated herein by reference). Examples of particular HAPs useful in the methods of the invention include without limitation TH-302. Methods of synthesizing, formulating, and using TH-302 and other HAPs are described in the various patent publications and applications referenced in the "Background of the Invention", above, which are incorporated herein by reference.

"Immunomodulators" refers to an activator of a costimulatory molecule, or an inhibitor of an immune-inhibitory molecule, or a vaccine. The Programmed Death 1 (PD-1) protein is an inhibitory member of the extended CD28/CTLA4 family of T cell regulators (Okazaki et al. (2002) Curr Opin Immunol 14: 391779-82; Bennett et al. (2003) J. Immunol. 170:711-8). PD-1 is expressed on activated B cells, T cells, and monocytes. PD-1 is an immune-inhibitory protein that negatively regulates TCR signals (Ishida, Y. et al. (1992) EMBO J. 11:3887-3895; Blank, C. et al. (Epub 2006 Dec. 29) Immunol. Immunother. 56(5):739-745), and is up-regulated in chronic infections. The interaction between PD-1 and PD-L1 can act as an immune checkpoint, which can lead to, e.g., a decrease in infiltrating lymphocytes, a decrease in T-cell receptor mediated proliferation, and/or immune evasion by cancerous or infected cells (Dong et al. (2003) J. Mol. Med. 81:281-7; Blank et al. (2005) Cancer Immunol. Immunother. 54:307-314; Konishi et al. (2004) Clin. Cancer Res. 10:5094-100). Immune suppression can be reversed by inhibiting the local interaction of PD-1 with PD-L1 or PD-L2; the effect is additive when the interaction of PD-1 with PD-L2 is blocked as well (Iwai et al. (2002) Proc. Nat'l. Acad. Sci. USA 99:12293-7; Brown et al. (2003) J. Immunol. 170:1257-66). Immunomodulation can be achieved by binding to either the immune-inhibitory protein (e.g., PD-1) or to binding proteins that modulate the inhibitory protein (e.g., PD-L1, PD-L2). In one embodiment, the combination therapies of the invention include an immunomodulator that is an inhibitor or antagonist of an inhibitory molecule of an immune checkpoint molecule. In another embodiment the immunomodulator binds to a protein that naturally inhibits the immuno-inhibitory checkpoint molecule. When used in combination with a HAP, these immunomodulators can enhance the anti-cancer response, and thus enhance efficacy relative to treatment with the anti-cancer compound alone.

The term "immune checkpoints" refers to a group of molecules on the cell surface of CD4 and CD8 T cells. These molecules can effectively serve as "brakes" to down-modulate or inhibit an adaptive immune response. Immune checkpoint molecules include, but are not limited to, Programmed Death 1 (PD-1) and Cytotoxic T-Lymphocyte Antigen 4 (CTLA-4), which directly inhibit immune cells.

Immunotherapeutic agents which can act as immune checkpoint inhibitors useful in the methods of the present invention, include, but are not limited to, inhibitors of PD-1, PD-L1, PD-L2, and CTLA4. Inhibition of an inhibitory molecule can be performed by inhibition at the DNA, RNA or protein level. In some embodiments, an inhibitory nucleic acid (e.g., a dsRNA, siRNA or shRNA), can be used to inhibit expression of an inhibitory molecule. In other embodiments, the inhibitor of an inhibitory signal is a polypeptide, e.g., a soluble ligand, or an antibody or antigen-binding fragment thereof, that binds to the inhibitory molecule.

"Patient" or "subject" refers to mammals, particularly humans, and so includes animals of veterinary and research interest, such as simians, cattle, horses, dogs, cats, and rodents with cancer or another hyperproliferative disease.

"Pharmaceutically acceptable salt" refers to pharmaceutically acceptable salts derived from a variety of organic and inorganic counter ions well known in the art that include, by way of example only, sodium, potassium, calcium, magnesium, ammonium, and tetraalkylammonium, and when the molecule contains a basic functionality, salts of organic or inorganic acids, such as hydrochloride, hydrobromide, tartrate, mesylate, acetate, maleate, and oxalate. Suitable salts include those described in Stahl and Wermuth (Eds.), Handbook of Pharmaceutical Salts Properties, Selection, and Use; 2002.

QnD or qnd refers to drug administration once every n days. For example, QD (or qd) refers to once every day or once daily dosing, Q2D (or q2d) refers to a dosing once every two days, Q7D refers to a dosing once every 7 days or once a week, Q5D refers to dosing once every 5 days.

"Reduction" of a symptom or symptoms (and grammatical equivalents of this phrase) refers to decreasing the severity or frequency of the symptom(s), or elimination of the symptom(s).

"Relapsed or refractory" refers to a type of cancer that is resistant to treatment with an agent, such an immunomodulator or a hypoxia activated prodrug, or responds to treatment with an agent but recurs with or without being resistant to that agent.

"Therapeutically effective amount" of a drug or an agent refers to an amount of the drug or the agent that, when administered to a patient with cancer or another hyperproliferative disease, will have the intended therapeutic effect, e.g., alleviation, amelioration, palliation or elimination of one or more manifestations of cancer or another hyperproliferative disease in the patient. A therapeutic effect does not necessarily occur by administration of one dose, and may occur only after administration of a series of doses. Thus, a therapeutically effective amount may be administered in one or more administrations.

"Treating" or "treatment of" a condition or patient refers to taking steps to obtain beneficial or desired results, including clinical results. For purposes of this invention, beneficial or desired clinical results include, but are not limited to, alleviation or amelioration of one or more symptoms of cancer or another hyperproliferative disease including conditional survival and reduction of tumor load or volume; diminishment of extent of disease; delay or slowing of disease progression; amelioration, palliation, or stabilization of the disease state; or other beneficial results.

The present invention arises in part from the discovery that the pharmacological activation of an immune checkpoint can potentiate the efficacy of TH-302 and other HAPs, when administered in accordance with the methods of the invention. As the examples below demonstrate, immunomodulators in combination with TH-302 show increased anti-cancer efficacy. Taken together, the results support that efficacious treatment of cancer can be achieved by administering one or more immunomodulators in combination with tumor-hypoxia targeting HAPs, such as, TH-302.

In certain embodiments, the HAPs described herein are administered in combination with one or more immunomodulators that are inhibitors of PD-1, PD-L1 and/or PD-L2 or an agonist of 4-1BB. Each such inhibitor or agonist may be an antibody, an antigen binding fragment thereof, an immunoadhesin, a fusion protein, or an oligopeptide. Examples of such immunomodulators are known in the art and described in more detail herein.

### Hypoxia activated drug administration

In one aspect, the present invention provides a method of treating cancer comprising administering a therapeutically effective amount of a HAP and a therapeutically effective amount of an immunomodulator to a patient in need of such treatment thereby treating the cancer. In one embodiment, the combination therapy is administered to a patient that has been previously treated with an immunomodulator or a HAP, but the cancer is progressing despite the therapy, or the therapy has been discontinued due to cancer progression. In other embodiments, the patient has not been previously treated with any anti-cancer drug. In other embodiments, the patient has been previously treated with an anti-cancer drug other than an immunomodulator or a HAP.

In one embodiment, the HAP is TH-302. Therapeutic dosages of TH-302 will likely be in the range of 1 mg to 3000 mg per day. The specific dose level selected for any particular patient will depend upon a variety of factors, including the activity of the specific compound employed, the age, body weight, general health, sex, diet, time of administration, route of administration, and rate of excretion, drug combination and the severity of the condition undergoing therapy.

In another aspect, the present invention provides a method of treating cancer, by administering a therapeutically effective amount of TH-302 in the range of less than about 320 mg/m² to a patient in need of such treatment. In another embodiment, the present invention provides a method of treating cancer, by administering about 320 mg/m² TH-302. In another embodiment, the present invention provides a method of treating cancer, by administering about 400 mg/m² TH-302. In one embodiment, the present invention provides a method of treating cancer, by administering about 420 mg/m² TH-302. Animal studies showing therapeutically effective administration of TH-302 with antitumor effect are described in the Examples below.

In one embodiment, the therapeutically effective amount of TH-302 is administered at a frequency of at least once per week, at least once per two weeks, up to once per month. In one embodiment, TH-302 is administered for a period of 1-40 weeks, at least 1 week, at least 2 weeks or up to at least 12 weeks. In other embodiments, longer periods of administration are employed.

Various frequencies and periods of TH-302 administration for effective cancer treatment according to the present methods are described in the Examples below.

Each of the above schedules can be considered a "cycle" of therapy. Patients will generally receive more than one cycle of therapy, although there may breaks of at least a day, and more generally a week or longer, between each cycle of therapy. Other HAPs are generally dosed in accordance with the above schedules and amounts, with the amount adjusted to reflect how active the compound is relative to TH-302.

When an immunomodulator is combined with a hypoxia activated prodrug according to the present invention, the immunomodulator is contemplated to be administered in amounts and dosing frequencies as disclosed herein below, or in amounts and frequencies apparent to the skilled artisan in view of this disclosure, or in amounts and frequencies approved by the FDA or other regulatory authority for use in the treatment of cancer.

In various embodiments, the patient's cancer treated is a metastatic cancer or a refractory and/or relapsed cancer that is refractory to first, second, or third line treatment. In another embodiment, the treatment is a first, a second, or a third line treatment. As used herein, the phrase "first line" or "second line" or "third line" refers to the order of treatment received by a patient. First line treatment regimens are treatments given first, whereas second or third line treatment are given after the first line therapy or after the second line treatment, respectively. Therefore, first line treatment is the first treatment for a disease or condition. In patients with cancer, primary treatment can be surgery, chemotherapy, radiation therapy, or a combination of these therapies. First line treatment is also referred to those skilled in the art as primary therapy or primary treatment. Typically, a patient is given a subsequent chemotherapy regimen because the patient did not show a positive clinical or only showed a sub-clinical response to the first line therapy, or the first line treatment has stopped. In this context, "chemotherapy" is used in its broadest sense to incorporate not only classic cytotoxic chemotherapy but also molecularly targeted therapies and immunotherapies.

In another aspect, the treatment methods of the present invention are used for treating hyperproliferative diseases other than cancer.

Methods of preparation of and pharmaceutical compositions of hypoxia activated prodrugs, and other methods of treating cancer by administering various HAPs are described in U.S. Patent Nos. 7,550,496; 8,299,088; 8,003,625; 8,507,464; 8,664,204; 9,226,932; 8,552,048; and 8,946,275 and U.S. Patent Application Nos. 13/806,088; 13/809,135; 14/009,068; 14/110,819; 14/033491; 14/367,188; 14/367,152; 14/375,417; 14/380,054; 14/783776; and 14/907,190 and PCT International Patent Application Nos. PCT/US2014/062532; PCT/US2015/029297; PCT/US2015/040642; PCT/US15/41100; and PCT/US2015/61248 each of which is incorporated herein by reference.

Other methods of treating cancers, which may be used in combination with the methods of the present invention, are known to one of skilled in the art, and are described, for example, in the product descriptions found in the 2010 or more current edition of the Physician's Desk Reference, Medical Economics Company, Inc., Oradell, NJ; Goodman and Gilman's The pharmacological basis of therapeutics., Eds. Hardman et al., McGraw-Hill. New York. (US) 2011, 12th Ed., and in publications of the U.S. Food and Drug Administration and the NCCN Guidelines (National Comprehensive Cancer Network). Such methods can be appropriately modified by one of skill in the art, in view of this disclosure, to practice the treatment methods of the present invention.

### Immunomodulators and their administration

The following compounds are useful for administration in combination with a HAP according to the present invention.

In some embodiments, the immunomodulator is an immunoadhesin (e.g., an immunoadhesin comprising an extracellular or PD-1 binding portion of PD-LI or PD-L2 fused to a constant region (e.g., an Fc region of an immunoglobulin sequence).

In some embodiments, the immunomodulator is a PD-1 inhibitor such as AMP-224.

In some embodiments, the immunomodulator is a PD-LI inhibitor such as anti-PD-LI antibody.

In some embodiments, the immunomodulator is an anti-PD-LI binding antagonist chosen from YW243.55.S70, MPDL3280A, MEDI-4736, MSB-0010718C, or MDX-1105. MDX-1105, also known as BMS-936559, is an anti-PD-LI antibody described in WO2007/005874. Antibody YW243.55.S70 is an anti-PD-LI described in WO 2010/077634.

In some embodiments, the immunomodulator is nivolumab (CAS Registry Number: 946414-94-4). Alternative names for nivolumab include MDX-1106, MDX-1106-04, ONO-4538, or BMS-936558. Nivolumab is a fully human IgG4 monoclonal antibody which specifically blocks PD-1. Nivolumab (clone 5C4) and other human monoclonal antibodies that specifically bind to PD-1 are disclosed in U.S. Pat. No. 8,008,449, EP2161336 and WO2006/121168.

In some embodiments, the immunomodulator is an anti-PD-1 antibody Pembrolizumab. Pembrolizumab (also referred to as Lambrolizumab, MK-3475, MK03475, SCH-900475 or KEYTRUDA.RTM.; Merck) is a humanized IgG4 monoclonal antibody that binds to PD-1. Pembrolizumab and other humanized anti-PD-1 antibodies are disclosed in Hamid, O. et al. (2013) New England Journal of Medicine 369 (2): 134-44, U.S. Pat. No. 8,354,509, WO2009/114335, and WO2013/079174.

In some embodiments, the immunomodulator is Pidilizumab (CT-011; Cure Tech), a humanized IgG1k monoclonal antibody that binds to PD1. Pidilizumab and other humanized anti-PD-1 monoclonal antibodies are disclosed in WO2009/101611.

Other anti-PD1 antibodies useful as immunomodulators for use in the methods disclosed herein include AMP 514 (Amplimmune), and anti-PD1 antibodies disclosed in U.S. Pat. No. 8,609,089, US 2010028330, and/or US 20120114649. In some embodiments, the anti-PD-L1 antibody is MSB0010718C. MSB0010718C (also referred to as A09-246-2; Merck Serono) is a monoclonal antibody that binds to PD-L1.

In some embodiments, the immunomodulator is MDPL3280A (Genentech/Roche), a human Fc optimized IgG1 monoclonal antibody that binds to PD-L1. MDPL3280A and other human monoclonal antibodies to PD-L1 are disclosed in U.S. Pat. No. 7,943,743 and U.S. Publication No.: 20120039906. Other anti-PD-L1 binding agents useful as immunomodulators for methods of the invention include YW243.55.570 (see WO2010/077634), MDX-1105 (also referred to as BMS-936559), and anti-PD-L1 binding agents disclosed in WO2007/005874.

In some embodiments, the immunomodulator is AMP-224 (B7-DCIg; Amplimmune; e.g., disclosed in WO2010/027827 and WO2011/066342), is a PD-L2 Fc fusion soluble receptor that blocks the interaction between PD1 and B7-H1.

In certain embodiments, the immunomodulator is an 4-1BB agonistic antibody.

In one embodiment, the immunomodulator used is a soluble ligand (e.g., a CTLA-4-Ig), or an antibody or antibody fragment that binds to PD-L1, PD-L2 or CTLA4. For example, the anti-PD-1 antibody molecule can be administered in combination with an anti-CTLA-4 antibody, e.g., ipilimumab, for example. Exemplary anti-CTLA4 antibodies include Tremelimumab (IgG2 monoclonal antibody available from Pfizer, formerly known as ticilimumab, CP-675,206); and Ipilimumab (CTLA-4 antibody, also known as MDX-010, CAS No. 477202-00-9).

In one embodiment, an anti-PD-1 antibody molecule is administered after treatment with a compound of the invention as described herein.

In another embodiment, an anti-PD-1 or PD-L1 antibody molecule is administered in combination with an antigen-binding fragment thereof. In another embodiment, the anti-PD-1 or PD-L1 antibody molecule is administered in combination with an antigen-binding fragment thereof. In yet other embodiments, the anti-PD-1 or PD-L1 antibody molecule is administered in combination with an antigen-binding fragments thereof. The combination of antibodies recited herein can be administered separately, e.g., as separate antibodies, or linked, e.g., as a bispecific or trispecific antibody molecule. In one embodiment, a bispecific antibody that includes an anti-PD-1 or PD-L1 antibody molecule, or antigen-binding fragment thereof, is administered. In certain embodiments, the combination of antibodies recited herein is used to treat a cancer, e.g., a cancer as described herein (e.g., a solid tumor). The efficacy of the aforesaid combinations can be tested in animal models known in the art. For example, the animal models to test the synergistic effect of anti-PD-1 are described, e.g., in Woo et al. (2012) Cancer Res. 72(4):917-27).

Exemplary doses of such immunomodulators that can be used in combination with the compounds of the invention include a dose of anti-PD-1 antibody molecule of about 1 to 10 mg/kg, e.g., 3 mg/kg, and a dose of an anti-CTLA-4 antibody, e.g., ipilimumab, of about 3 mg/kg.

Examples of embodiments of the methods of using the HAP of the invention in combination with an immunomodulator include these:
i. A method to treat cancer in a subject, comprising administering to the subject a HAP as described herein, and an immunomodulator.
ii. The method of any of embodiments i-iii above, wherein the inhibitor of the immune checkpoint molecule is chosen from PD-1, PD-L1, PD-L2, and CTLA4.
iii. The method of any of any of embodiments i-ii, wherein the inhibitor of the immune checkpoint molecule is chosen from an inhibitor of PD-1, PD-L1, or CTLA4, or any combination thereof.
iv. The method of any of embodiments i-iii, wherein the inhibitor of the immune checkpoint molecule is a soluble ligand or an antibody or antigen-binding fragment thereof, that binds to the immune checkpoint molecule.
v. The method of any of embodiments i-iv, wherein the antibody molecule is a bispecific or multispecific antibody molecule that has a first binding specificity to PD-1 or PD-L1 and a second binding specificity to PD-L2.
vi. The method of any of embodiments i-v, wherein the immunomodulator is an anti-PD-1 antibody chosen from Nivolumab, Pembrolizumab or Pidilizumab.
vii. The method of any of embodiments i-vi, wherein the immunomodulator is an anti-PD-L1 antibody chosen from YW243.55.570, MPDL3280A, MEDI-4736, MSB-0010718C, or MDX-1105.
viii. The method of any of embodiments i-vii, wherein the immunomodulator is an anti-PD-1 antibody molecule administered by injection (e.g., subcutaneously or intravenously) at a dose of about 1 to 30 mg/kg, e.g., about 5 to 25 mg/kg, about 10 to 20 mg/kg, about 1 to 5 mg/kg, or about 3 mg/kg, e.g., once a week to once every 2, 3, or 4 weeks.
ix. The method of embodiment viii, wherein the anti-PD-1 antibody molecule is administered at a dose from about 10 to 20 mg/kg every other week.
x. The method of embodiment ix, wherein the anti-PD-1 antibody molecule, e.g., nivolumab, is administered intravenously at a dose from about 1 mg/kg to 3 mg/kg, e.g., about 1 mg/kg, 2 mg/kg or 3 mg/kg, every two weeks.
xi. The method of embodiment x, wherein the anti-PD-1 antibody molecule, e.g., nivolumab, is administered intravenously at a dose of about 2 mg/kg at 3-week intervals.

Thus, in accordance with the present invention, TH-302 or another HAP is co-administered with an immunomodulator, optionally in combination with other treatments. A synergistic effect may be achieved by using more than one compound in a pharmaceutical composition of the invention, i.e. a HAP is combined with at least another agent as active ingredient, which is either another HAP, or an immunomodulator, or both, or another anti-cancer agent. The active ingredients useful in the methods of the invention can be used either simultaneously (as in an admixed formulation) or sequentially. Thus, the invention also relates to a compound or pharmaceutical composition for inhibiting abnormal cell growth or cancer in a mammal which comprises an amount of a HAP, or a pharmaceutically acceptable salt or solvate or prodrug thereof, in combination with an amount of another immunomodulator, wherein the amounts of the compound, salt, solvate, or prodrug, and of the immunomodulator are together effective in inhibiting abnormal cell growth or cancer in a patient. The combination therapies described herein are thus suitable for use in combination with known anti-cancer agents.

The invention also relates to a set of items, which may be packaged into a kit, consisting of separate packs of an effective amount of a HAP and an immunomodulator (or pharmaceutically acceptable salts, derivatives, solvates, and stereoisomers thereof, including mixtures thereof in all ratios, and optionally an effective amount of a further medicament active ingredient). The set or kit comprises suitable containers, such as boxes, individual bottles, bags, or ampoules. The set may, for example, comprise separate ampoules, each containing an effective amount of a HAP and an immunomodulator (or pharmaceutically acceptable salts, derivatives, solvates, and stereoisomers thereof, including mixtures thereof in all ratios, and optionally an effective amount of a further medicament active ingredient), each in dissolved or lyophilized form. The set or kit of the invention may also contain an article that contains written instructions or directs the user to written instructions that explain the how the compounds are administered in accordance with the invention to treat a disease, such as cancer.

The invention also relates to the use of HAPs and immunomodulator compounds and/or physiologically acceptable salts thereof for the prophylactic or therapeutic treatment and/or monitoring of diseases, such as cancer, that are caused, mediated, and/or propagated by abnormal cellular proliferative activity.

Furthermore, the invention relates to the use of HAPs and immunomodulators thereof for the production of a medicament for the prophylactic or therapeutic treatment and/or monitoring of diseases, such as cancer, that are caused, mediated, and/or propagated by abnormal cellular proliferative activity.

HAPs and immunomodulators and/or a physiologically acceptable salt thereof can also be employed as intermediates for the preparation of further medicament active ingredients. The medicament is preferably prepared in a non-chemical manner, e.g. by combining the active ingredient with at least one solid, fluid and/or semi-fluid carrier or excipient, and optionally in conjunction with a single or more other active substances in an appropriate dosage form.

Another object of the present invention are HAPs and immunomodulators according to the invention and/or physiologically acceptable salts thereof for use in the prophylactic or therapeutic treatment and/or monitoring of diseases, such as cancer, that are caused, mediated, and/or propagated by abnormal cellular proliferative activity. Another preferred object of the invention concerns HAPs and immunomodulators according to the invention and/or physiologically acceptable salts thereof for use in the prophylactic or therapeutic treatment and/or monitoring of hyperproliferative disorders, including cancer.

The prior teaching of the present specification concerning HAPs and immunomodulators, including any preferred embodiment thereof, is valid and applicable without restrictions to the HAPs and immunomodulators and their salts for use in the prophylactic or therapeutic treatment and/or monitoring of hyperproliferative disorders.
The invention will now be described by the following numbered clauses which are not claims:
1. A method of treating cancer, said method comprising administering to a patient in need of such treatment a therapeutically effective amount of a hypoxia-activated prodrug (HAP) in combination with a therapeutically effective amount of an immunomodulator.
2. A method of treating cancer, comprising the steps of: (i) identifying patients with cancer who are or would be unresponsive to immunotherapy alone; and (ii) administering a therapeutically effective amount of a hypoxia-activated prodrug (HAP) in combination with a therapeutically effective amount of an immunomodulator only to patients who are identified as who are or would be unresponsive to immunotherapy alone.
3. The method of clause 1 or 2, wherein said patient is human.
4. The method of clause 3, wherein said compound is TH-302.
5. The method of any one of clauses 1 to 4, wherein said immunomodulator is selected from the group consisting of a CTLA-4 antibody and a PD-1 antibody.
6. The method of clause 5, wherein said immunomodulator is selected from the group consisting of ipilimumab, pembrolizumab and nivolumab.
7. The method of clause 6, wherein said immunomodulator is ipilimumab.
8. The method of clause 6, wherein said immunomodulator is pembrolizumab.
9. The method of clause 6, wherein said immunomodulator is nivolumab.
10. The method of any one of clauses 4 to 9 wherein the TH-302 is administered in a dose of less than about 320 mg/m².
11. The method of any one of clauses 4 to 9 wherein the TH-302 is administered for one or more dosage cycles, each cycle comprising an infusion of TH-302 in the range of about 320 mg/m² or 400 mg/m² to about 480 mg/m².
12. The method of clause 10 or 11 wherein the TH-302 is administered over an infusion period of about 1 to about 6 hours.
13. The method of clause 12 wherein the TH-302 is administered once on days 1, 8, 29 and 36 of a 5 week cycle.
14. The method of clause 13 wherein the immunomodulator is ipilimumab and administered for one or more dosage cycles, each cycle comprising an infusion of ipilimumab in a unit dose of about 3 mg/kg over an infusion period of about 1 to 2 hours once every three weeks of a 12 week cycle.
15. The method of any one of clauses 3 to 14 wherein the immunomodulator is administered on a different day than the administration of TH-302.
16. The method of clause 14 wherein the immunomodulator is administered on the same day as the administration of TH-302.
17. The method of clause 16 wherein the TH-302 is administered prior to the administration of the immunomodulator if administered on the same day.
18. The method of clause 16 wherein the TH-302 is administered after the administration of the immunomodulator if administered on the same day.
19. A pharmaceutical combination comprising: a) a dose of TH-302, formulated for infusion in the range of less than about 320 mg/m²; and b) a dose of a immunomodulator.
20. A pharmaceutical combination comprising: a) a dose of TH-302, formulated for infusion in the range of about 320 mg/m² to about 480 mg/m²; and b) a dose of ipilimumab formulated for infusion of about 3 mg/kg.
21. The pharmaceutical combination of clause 19 or 20, packaged together.
22. The method or combination of any one of the preceding clauses wherein the cancer is selected from the group consisting of prostate cancer, pancreatic cancer, melanoma and head and neck cancer.
23. The method or combination of clause 22 wherein the head and neck cancer is Human Papillomavirus (HPV) negative head and neck cancer.
24. The pharmaceutical combination of clause 19 or 20, wherein the packaged pharmaceutical combination includes instructions for use of the combination in treating a cancer selected from the group consisting of prostate cancer, pancreatic cancer, melanoma and head and neck cancer.

The following examples illustrate various aspects and embodiments of the invention.

### EXAMPLES

### Example 1. Enhancement of TH-302's antitumor activity by immune modulating agents in mice xenografts.

5 x 10⁵ to 1 × 10⁶ TRAMP-C2, C57BL/6J, B6, FVB F1 cells and/or Panc02 pancreatic ductal adenocarcinoma cells expressing luciferase are orthotopically implanted s.c. on the right flank of albino or ODD-Luc reporter mice. The mice are treated with TH-302 alone (50 mg/kg i.p., every day for 5 days), either 1 week, 3 weeks or 16 weeks after TRAMP cell implantation, with a week of rest in between. Some mice are also treated with CTLA-4 blocking antibody 9H10, PD-1 blocking antibody RMP1-14 and/or agonistic 4-1BB antibody given i.p. on days 1, 4, 7 and days 13, 16 and 19 for one to three cycles. Mice are monitored for survival (log-rank, mantel-cox test) and tumor growth was observed by IVIS once a week up to 140 days after tumor implantation. The mice were then sacrificed and their organs were weighed and tumors were isolated, fixed in paraformaldehyde or acetone, embedded/mounted in OCT, frozen, sectioned, stained for hypoxia with anti-piminidazole FITC, GR-1 V450, CD11b Alexa 546, and/or F4/80 Alexa 647. The data demonstrates that TH-302 alone or with an immunomodulator alone produced a growth delay, and that the combination of an immunomodulator with TH-302 significantly prolonged the time required for tumor volume doubling relative to either drug alone.

These examples demonstrate that TH-302 activity is significantly enhanced by co-administration with an immunomodulator; and that co-administration of an immunomodulator significantly enhances TH-302-mediated anti-tumor activity in xenografts.

The data presented in these examples support a new approach for the treatment of cancer in which an immunomodulator is administered in combination with a HAP such as TH-302 to provide more efficacious therapy.

It should be understood that although the present invention has been specifically disclosed by certain aspects, embodiments, and optional features, modification, improvement and variation of such aspects, embodiments, and optional features can be resorted to by those skilled in the art, and that such modifications, improvements and variations are considered to be within the scope of this disclosure.

The invention has been described broadly and generically herein. Each of the narrower species and subgeneric groupings falling within the generic disclosure also form part of the invention. In addition, where features or aspects of the invention are described in terms of Markush groups, those skilled in the art will recognize that the invention is also thereby described in terms of any individual member or subgroup of members of the Markush group.

## Claims

1. A hypoxia activated prodrug (HAP) for use in a method of treating cancer, wherein the HAP is administered to a patient in combination with an anti-PD-1 antibody and an anti-CTLA-4 antibody; and wherein the HAP is TH-302.

2. A HAP for use according to claim 1, wherein the cancer is prostate cancer.

3. A HAP for use according to claim 1, wherein the cancer is pancreatic cancer.

4. A HAP for use according to claim 1, wherein the cancer is head and neck cancer.

5. A HAP for use according to claim 4, wherein the head and neck cancer is Human Papillomavirus (HPV) negative head and neck cancer.

6. A HAP for use according to any one of the preceding claims, wherein the cancer is a metastatic cancer.

7. A HAP for use according to any one of the preceding claims, wherein the anti-CTLA4 antibody is ipilimumab.

8. A HAP for use according to any one of the preceding claims, wherein the anti-PD-1 antibody is pembrolizumab.

9. A HAP for use according to any one of claims 1 to 7, wherein the anti-PD-1 antibody is nivolumab.

10. A HAP for use according to any one of the preceding claims, wherein the cancer that is refractory to a first, second, or third line treatment received by the patient.

11. A HAP for use according to any one of the preceding claims, wherein the HAP in combination with the immunomodulators is administered to a patient that has been previously treated with an immunomodulator or a HAP, but the cancer is progressing despite the therapy, or the therapy has been discontinued due to cancer progression.

12. A HAP for use according to any one of the preceding claims, wherein the HAP is administered to the patient in a dose of less than about 320 mg/m².

13. A HAP for use according to any one of the preceding claims, wherein the HAP is administered to the patient by infusion in a dose of about 320 mg/m² to about 480 mg/m².
